# EUROPÄISCHE PATENTANMELDUNG

(11) **EP 2 770 451 A1**
(43) Veröffentlichungstag der Anmeldung: **27.08.2014**
(21) Anmeldenummer: 14155905.4
(22) Anmeldetag: 20.02.2014
(51) Int. Cl.: G06F 19/00, G06Q 50/20

(54) **Verfahren zur Vermittlung von einzelfallbezogenen, institutionsübergreifenden Behandlungsschritten**

(30) Priorität: 21.02.2013 DE 102013202866
(71) Anmelder: soleosoft GmbH, 07743 Jena (DE)
(72) Erfinder: Steffen Jugel, 37345 Bischofferode (DE); Andreas Rudolph, 07751 Jena (DE)
(74) Vertreter: Kruspig, Volkmar

(57) **Zusammenfassung**

Die Erfindung betrifft ein Verfahren und eine Anordnung zur Vermittlung von einzelfallbezogenen, institutionsübergreifenden Behandlungsschritten bei einer aus einer Vielzahl von individuellen Einzelpatienten bestehenden Patientenmenge, unter Nutzung eines Kommunikationsnetzwerkes mit einer verteilten Endgerätemenge und einer zentralen Servereinheit. Verfahrensseitig werden folgende Verfahrensschritte ausgeführt: Erfassen fall- und personenspezifischer Einzeldaten an mindestens einem ersten Endgerät mittels eines auf dem Endgerät betriebenen Falleingangsmoduls, Erzeugen einer Fallanfrage durch das Falleingangsmodul und übermitteln der Fallanfrage an die zentrale Servereinheit, Übergeben der Fallanfrage an einen von der zentralen Servereinheit betriebenen Fallvermittlungsalgorithmus, Ausgabe der Fallanfrage von der zentralen Servereinheit an mindestens ein durch den Fallvermittlungsalgorithmus ausgewähltes zweites Endgerät, Verarbeiten der Fallanfrage auf dem mindestens einen zweiten Endgerät in einem dort betriebenen Fallausgangsmodul, Rückleiten einer Fallantwort von dem zweiten Endgerät an die zentrale Servereinheit, Weiterleiten der Fallantwort von der zentralen Servereinheit auf das erste Endgerät.

## Beschreibung

Die Erfindung betrifft ein Verfahren zur Vermittlung von einzelfallbezogenen, institutionsübergreifenden Behandlungsschritten bei einer aus eine Vielzahl von individuellen Einzelpatienten bestehenden Patientenmenge nach dem Oberbegriff des Anspruchs 1.

Die Behandlung medizinischen Einzelfällen, die ein interdisziplinäres Zusammenwirken verschiedener Institutionen erfordern, nimmt im Zuge einer steigenden Lebenserwartung und mit dem Fortschritt der medizinischen Behandlungsmethoden zu.

Derartige Einzelfälle erfordern einen genau aufeinander abgestimmten Zeitplan aus Behandlungsvorbereitung, Behandlungsausführung, aus Nachsorge und aus Rehabilitation. Ein Beispiel hierfür ist beispielsweise die moderne Krebstherapie aus vorbereitender Bestrahlung oder Chemotherapie, anschließender Operation, postoperativer Versorgung, erneuter Chemotherapie und anschließender Rehabilitation mit einer abschließenden, sich teilweise über Jahre erstreckender Nachkontrolle.

Dabei besteht das Problem, dass einerseits beträchtliche Patientenzahlen durch die behandelnden Institutionen geführt werden müssen, während andererseits aber jeder Patient einen individuellen Einzelfall darstellt, der auch individuell versorgt werden muss. Dabei muss einerseits die genaue Behandlungsplanung jedes Patienten berücksichtigt werden, sodass kein Behandlungsschritt ausgelassen wird, andererseits dürfen nach Möglichkeit keine langen Wartezeiten entstehen. Außerdem ist es erstrebenswert, dass alle medizinischen Institutionen optimal ausgelastet sind, denn diese sind nicht zuletzt auch betriebswirtschaftlichen Kriterien unterworfen.

Es zeigt sich somit, dass hier ein ganzer Problemkomplex vorliegt, der sich in individuellen Einzelabstimmungen zwischen Patient und Arzt, Patient und Klinik oder Klinik und Arzt praktisch nicht lösen lässt. Die dabei notwendigen einzelnen Vermittlungen und Absprachen sind zeit- und personalintensiv, sie verursachen zusätzliche Kosten und gehen außerdem zulasten des Patienten. Es existieren keine Mittel, mit dem sich ein solcher Vermittlungsprozess selbsttätig und automatisiert ausführen lässt.

Es besteht somit die Aufgabe, Verfahren und Mittel anzugeben, mit dem sich der zeitraubende und personalintensive Vermittlungsvorgang vollständig beseitigen lässt und also ohne menschlichen Ressourceneinsatz ausgeführt werden kann. Es soll dabei insbesondere möglich sein, eine möglichst große Patientenzahl zu handhaben und jedem einzelnen Patienten eine möglichst individuelle Behandlungsplanung zuteil werden zu lassen.

Die Aufgabe wird mit einem Verfahren zur Vermittlung von einzelfallbezogenen, institutionsübergreifenden Behandlungsschritten bei einer aus einer Vielzahl von individuellen Einzelpatienten bestehenden Patientenmenge gelöst. Das Verfahren läuft unter der Nutzung eines Kommunikationsnetzwerkes mit einer verteilten Endgerätemenge und einer zentralen Servereinheit ab, wobei erfindungsgemäß folgende Verfahrensschritte ausgeführt werden:

Es erfolgt zunächst ein Erfassen fall- und personenspezifischer Einzeldaten an einem ersten Endgerät mittels eines auf dem Endgerät betriebenen Falleingangsmoduls. Anschließend erfolgt ein Erzeugen einer Fallanfrage durch das Falleingangsmodul und ein Übermitteln der Fallanfrage an die zentrale Servereinheit. Die Fallanfrage wird dort an einen auf der zentralen Servereinheit betriebenen Fallvermittlungsalgorithmus übergeben. Als nächstes erfolgt eine Ausgabe der Fallanfrage von der zentralen Servereinheit an mindestens ein durch den Fallvermittlungsalgorithmus ausgewähltes zweites Endgerät. Auf dem mindestens einen zweiten Endgerät erfolgt dann ein Verarbeiten der Fallanfrage in einem dort betriebenen Fallausgangsmodul. Im Anschluss daran erfolgt ein Rückleiten einer Fallantwort von dem zweiten Endgerät an die zentrale Servereinheit. Abschließend erfolgt ein Übertragen der Fallantwort von der zentralen Servereinheit auf das mindestens eine erste Endgerät.

Grundgedanke des Verfahrens ist es somit, ein Kommunikationsnetz aus einer verteilten Endgerätemenge und einer zentralen Servereinheit dazu zu nutzen, um Fallanfragen erstens auf mindestens einem ersten Endgerät zu erzeugen und weiter zu leiten, diese Fallanfragen auf einem zentralen Vermittlungsserver zu vermitteln und im Ansprechen darauf Fallantworten zurück zu leiten. Die zentrale Servereinheit übernimmt dabei eine Vermittlungsfunktion. Die Fallanfrage wird dort erfasst und anhand der darin enthaltenen Daten erzeugt ein Fallermittlungsalgorithmus in einer automatisierten Weise das Weiterleiten der Fallanfrage an die übrigen Endgeräte.

Bei einer vorteilhaften Ausgestaltung sind das Falleingangsmodul und das Fallausgangsmodul gleichartig auf jedem Endgerät als jeweils ein Fallmodul ausgebildet. Das Fallmodul wird jeweils über ein Ein- und Ausgabeuntermodul für einen Nutzer und ein Anfrageuntermodul und ein Antwortuntermodul in Kommunikation mit der zentralen Servereinheit betrieben.

In einer solchen Ausgestaltung dient jedes Endgerät sowohl als Anfragestation als auch als Antwortstation. Dadurch wird eine echte bidirektionale Informationsvermittlung über das Kommunikationsnetz möglich.

Bei einer Ausführungsform erfolgt bei dem Antwortuntermodul über mindestens eine Absageausgang und einen Zusagenausgang ein Ausgeben einer positiven oder negativen Antwort auf die eingehende Fallanfrage. Das Antwortuntermodul lässt im einfachsten Falle nur zwei Antworttypen zu: Zusage oder Absage in Bezug auf den Inhalt der eingehenden Fallanfrage. Dadurch wird eine möglichst präzise Formulierung der Fallanfrage und auch der Fallantwort erzwungen, die bei der entgegennehmenden Stelle auch entsprechend geprüft und eindeutig beantwortet werden kann. Außerdem bietet dies auch die Möglichkeit, die über das Untermodul ausgegebenen Fallantworten je nach Zu- oder Absage zu protokollieren.

Bei einer weiteren Ausführungsform führt der auf der zentralen Servereinheit ablaufende Fallvermittlungsalgorithmus über einen Fallablaufsalgorithmus einen Zugriff auf eine Falldatenbank aus, wobei dadurch eine auf einen jeweiligen Einzelfall bezogene automatisierte Ablaufplanung der Folge von einzelnen Behandlungsschritte ausgeführt wird. Bei dieser Ausgestaltung wird die Fallvermittlung mit einer automatisierten Behandlungsplanung verknüpft. Die zentrale Servereinheit speichert dabei einerseits Patientendaten und andererseits grundlegende Behandlungsschritte, die in einer festgelegten Reihenfolge ausgeführt werden müssen. Sie sichert dadurch, dass keine Behandlungsschritte ausgelassen werden und somit unnötige und falsche Vermittlungen vorgenommen werden.

Bei einer zweckmäßigen Ausgestaltung wird jedes Falleingangsmodul, Fallausgangsmodul und/oder Fallmodul über einen Registrierungsvorgang an der zentralen Servereinheit angemeldet, wobei eine wahlweise Sperrung und/oder Inaktivierung jedes der Module ausführbar ist. Eine derartige Vorgehensweise erlaubt eine Kontrolle darüber, welche Nutzer das Verfahren vom welchem Endgerät aus in Anspruch nehmen und ermöglicht damit eine Zertifizierung und Qualitätssicherung.

In Verbindung damit kann weiterhin vorgesehen sein, dass die zentrale Servereinheit einen Datenbankzugriff auf Vertragsdaten, Sicherheitsdaten, Archivierungsdaten und dergleichen weitere Daten ausführt.

Bei einer weiteren Ausführungsform werden das Falleingangsmodul, das Fallausgangsmodul und/oder das Fallmodul als Bestandteile eines externen Fallmanagementsystems betrieben. Dabei führt das Fallmanagementsystem ein automatisiertes Aktivieren des jeweiligen Falleingangsmoduls, des Fallausgangsmoduls und/oder des Fallmoduls aus. Eine derartige Vorgehensweise stellt eine Ergänzung zu dem Fallvermittlungs- und/oder dem Fallablaufsalgorithmus auf der zentralen Servereinheit dar. Das Fallmanagementsystem greift aber hier an der Peripherie ein und ist auf einen speziellen lokalen Ablauf, beispielsweise Krebsbehandlung und -nachsorge in einer Klinik, eingerichtet.

Anordnungsseitig ist zur Vermittlung von auf einen jeweiligen Einzelfall bezogenen, institutionsübergreifenden Behandlungsschritten bei einer aus einer Vielzahl von individuellen Einzelpatienten bestehenden Patientenmenge ein Kommunikationsnetzwerk mit einer verteilten Endgerätemenge und einer zentralen Servereinheit vorgesehen. Dabei sind auf jedem Endgerät der Endgerätemenge programmtechnische Mittel in Form mindestens eines Fallmoduls und programmtechnische Mittel für einen auf der zentralen Servereinheit ablauffähigen Fallvermittlungsalgorithmus zum Eingeben, Weiterleiten und Vermitteln einer individuellen medizinischen Fallanfrage zwischen verschiedenen Anbietern und Behandlungsstationen vorgesehen.

Das erfindungsgemäße Verfahren soll nachfolgend anhand von Ausführungsbeispielen näher erläutert werden. Zur Verdeutlichung dienen die beigefügten Figuren 1 bis 7. Es werden für gleiche und/oder gleichwirkende Komponenten oder Verfahrensschritte dieselben Bezugszeichen verwendet.

Es zeigt:
- Fig. 1: einen grundsätzlichen Ablauf einer Fallvermittlung,
- Fig. 2: ein beispielhaftes Fallmodul im schematischen Aufbau,
- Fig. 3: ein beispielhaftes Zusammenwirken zwischen einem Fallvermittlungsalgorithmus, einem Fallablaufsalgorithmus und einer Falldatenbank,
- Fig. 4: einen beispielhaften Gesamtablaufplan bei der Ausführung des erfindungsgemäßen Verfahrens,
- Fig. 5: eine Übersichtsdarstellung des Verfahrensablaufs im Zusammenhang im Zusammenhang mit problembezogenen Einzelfallmanagementsystemen,
- Fig. 6: eine detaillierte Darstellung des Verfahrensablaufs gemäß Fig. 4 am Beispiel eines Krebstherapiemanagements auf der Anfragenseite,
- Fig. 7: eine detaillierte Darstellung des Verfahrensablaufs gemäß Fig. 4 am Beispiel des Krebstherapiemanagement auf der Antwortseite.

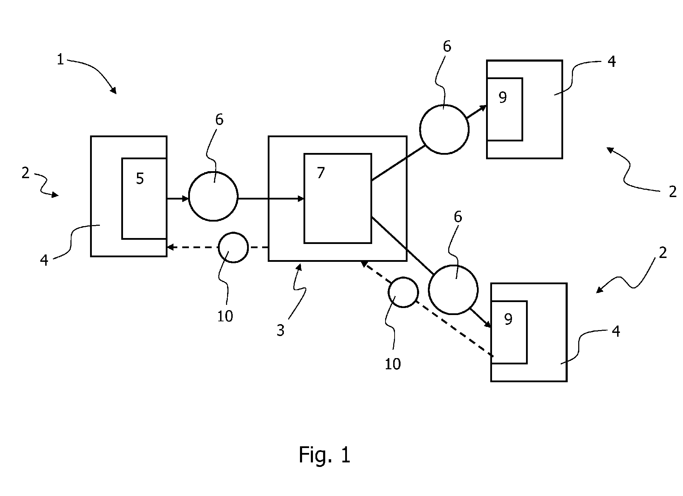

Fig. 1 zeigt einen grundsätzlichen Ablauf einer Fallvermittlung. Die Fallvermittlung wird innerhalb eines Kommunikationsnetzwerkes 1 ausgeführt. Dieses Kommunikationsnetzwerk ist insbesondere das Internet, es kann aber auch jedes andere, insbesondere drahtlos arbeitende Kommunikationsnetzwerk sein. Es kommuniziert dabei eine verteilte Endgerätemenge 2. Dabei werden Daten zwischen den einzelnen Endgeräte über eine zentrale Servereinheit 3 vermittelt. Die Endgeräte bilden die Orte, an welchem Nutzereingaben und Nutzerausgaben stattfinden. Die zentrale Servereinheit dient neben ihrer Vermittlungsfunktion außerdem als eine zentrale Datenbank zum Sammeln von einzelfallbezogenen Daten.

Im hier vorliegenden Beispiel ist ein erstes Endgerät 4. Im Wesentlichen erfolgt das Vermittlungsverfahren in der Weise, dass an dem ersten Endgerät 4 der verteilten Endgerätemenge fall- und personenspezifische Einzeldaten erfasst werden. Diese können insbesondere Falldaten eines Patienten sein, beispielsweise dessen Krankengeschichte, bisher erfolgte Behandlungsschritte, Medikamentierungen, aufgetretene Komplikationen und dergleichen Daten mehr. Die Erfassung dieser Daten erfolgt mittels eines Falleingangsmoduls 5 auf dem ersten Endgerät 4. Das Falleingangsmodul ist entweder hardwareartig auf dem ersten Endgerät 4 ausgebildet oder kann dort ein Form eines Programmcodes vorliegen. Dass Falleingangsmodul ist zweckmäßigerweise so ausgebildet, dass es den eingebenden Nutzer dazu zwingt, die spezifischen Einzeldaten möglichst detailliert und vollständig einzugeben. Hierzu können in dem Falleingangsmodul logische Abhängigkeitsprüfungen vorgesehen sein, die die eingegebenen Daten auf logische Fehler und Unstimmigkeiten prüfen. Weiterhin können Abstufungen im Sinne von Pflichtangaben und fakultativen Angaben für die Einzeldaten, sowie Kommentarfelder vorgesehen sein. Zweckmäßig ist dabei vor allem eine Gestaltung des Falleingangsmoduls, bei der auf dem ersten Endgerät eine für den Nutzer leicht verständliche, insbesondere graphische Nutzeroberfläche erzeugt wird.

Das Falleingangsmodul ist bei der zentralen Servereinheit 3 registriert und authentifiziert. Dadurch ist gesichert, dass eine Kontrolle darüber ausgeübt werden kann, welche Endgeräte mit welchem Falleingangsmodul an den Vermittlungsprozessen teilnehmen können und welche nicht. Die Kriterien dafür sind beispielsweise konsistente persönliche Daten der Endgerätenutzer, seriöse Handhabung der Verfahrensabläufe, Sicherheits- und Datenschutzaspekte oder regelmäßige Registrierungsabläufe in Verbindung mit Zertifizierungen.

Das Falleingangsmodul generiert in einem weiteren Schritt eine Fallanfrage 6. Die Fallanfrage fasst einerseits die vom Falleingangsmodul erfassten spezifischen Daten zusammen und dient somit zum einen dazu, die erfassten Einzelfalldaten an die zentrale Servereinheit und die zu vermittelnden weiteren Endgeräte zu übertragen. Wichtiger ist jedoch, dass in der Fallanfrage eine konkrete Anforderung so codiert ist, dass diese in einem automatischen Prozess angenommen oder abgelehnt werden kann. Diese Anforderung ist beispielsweise als Frage mit Nebenbedingungen formuliert und hat beispielsweise als Datensatz folgende Form:
Frage: Bettplatz - Rehabilitation - Unfall?
Nebenbedingung 1: schwere Beckenfraktur
Nebenbedingung 2: älter als 60 Jahre
Nebenbedingung 3: Herz-Kreislauf-Probleme

Sowohl die Frage als auch die Nebenbedingungen werden nun in der zentralen Servereinheit 3 einem Fallvermittlungsalgorithmus 7 übergeben. Dieser ordnet zunächst die einlaufende Fallanfrage kategorial ein. Diese Kategorisierung kann beispielsweise anhand der Begriffe "Bettplatz", "Rehabilitation" und "Unfall" vorgenommen werden. Dadurch wird die Fallanfrage bei der nachfolgenden Weiterleitung ausschließlich an solche Endgeräte weitergeleitet, die mit Institutionen verknüpft sind, die im Rehabilitationsbereich und speziell dort in der stationären Unfallnachsorge aktiv sind.

Der Fallvermittlungsalgorithmus leitet als nächstes die Fallanfrage über das Kommunikationsnetz an mehrere entsprechend ausgewählte zweite Endgeräte 8 weiter. Die Endgeräte 8 befinden sich zweckmäßigerweise an den Orten, die für den weiteren Ablauf des Behandlungsvorgangs von Bedeutung sind, beispielsweise in entsprechenden Kliniken, Praxen, Fahrdiensten und dergleichen Institutionen mehr. Jedes dieser zweiten Endgeräte enthält ein Fallausgangsmodul 9. Das Fallausgangsmodul 9 verarbeitet die einkommende Fallanfrage 6 und gibt im Ergebnis eine Fallantwort 10 aus.

Die Verarbeitung der Fallanfrage erfolgt beispielsweise so, dass in einem ersten Schritt aus der Fallanfrage zum einen der Inhalt der Fallanfrage ermittelt wird. In dem hier vorliegenden Beispiel ist dies die Kombination "Bettplatz - Rehabilitation - Unfall". Dadurch ist die Information codiert, dass ein Bettplatz für eine Rehabilitation eines Unfallpatienten gesucht wird. In einem zweiten Schritt werden durch das Fallausgangsmodul die mit der Fallanfrage verknüpften Nebenbedingungen analysiert und mit den auch in Datenform bereitstehenden Ressourcen der jeweiligen Institution, beispielsweise der entsprechenden Rehaklinik, abgeglichen. Es erfolgt somit ein Matching zwischen den Parametern der Fallanfrage einerseits und den Ressourcen der Institution andererseits.

Eine Fallantwort 10 wird dann durch das zweite Endgerät im Ergebnis dieses Matchings erzeugt. Sie beschränkt sich im einfachsten Fall auf die Wahl zwischen drei Antwortparametern: Zusage, Absage oder Warten. Der Antwortparameter der Zusage wird ausgegeben, wenn ein größtmögliches Matching zwischen den Parametern der Fallanfrage und den Ressourcen der Institution besteht. Der Antwortparameter der Absage wird beispielsweise ausgegeben, wenn mindestens in einem Punkt der Fallanfrage für einen gegebenen Zeitraum kein Matching zwischen den Parametern der Fallanfrage und den Ressourcen der Institution erreichbar ist. Der Antwortparameter des Wartens wird ausgegeben, wenn zum aktuellen Zeitpunkt kein Matching besteht, aber nach einem definierten Zeitraum erreicht werden kann.

Die Fallantwort 10 wird in einem darauf folgenden Schritt über das Kommunikationsnetz von dem zweiten Endgerät 8 zu dem ersten Endgerät 4 zurückgeleitet. Die zentrale Servereinheit 7 dient hier wieder als zentrale Vermittlungsstelle, die im einfachsten Fall sowohl die Fallanfrage als auch die Fallantwort protokolliert und in einer internen Datenbank abspeichert. Das Endgerät 4 führt dann regelmäßig wiederkehrende Abfragen an der zentralen Servereinheit 3 aus und ruft die dort anliegenden Fallantworten ab.

In dem ersten Endgerät 4 wird die Fallantwort schließlich in einer für einen Nutzer verständlichen Form angezeigt. Diese besteht beispielsweise aus der Angabe: "Rehabilitationsklinik X ist bereit zur Patientenaufnahme für Patient Y ab Datum Z".

Es ist einsichtig, dass sowohl auf dem Endgerät 4 als auch auf dem Endgerät 8 sowohl Falleingangs, als auch Fallausgangsmodule vorhanden sein können. Dadurch können von jedem Endgerät sowohl Fallanfragen als auch Fallantworten erzeugt werden und beide Endgeräte nehmen dadurch innerhalb des Verfahrensablaufs eine symmetrische Rolle ein.

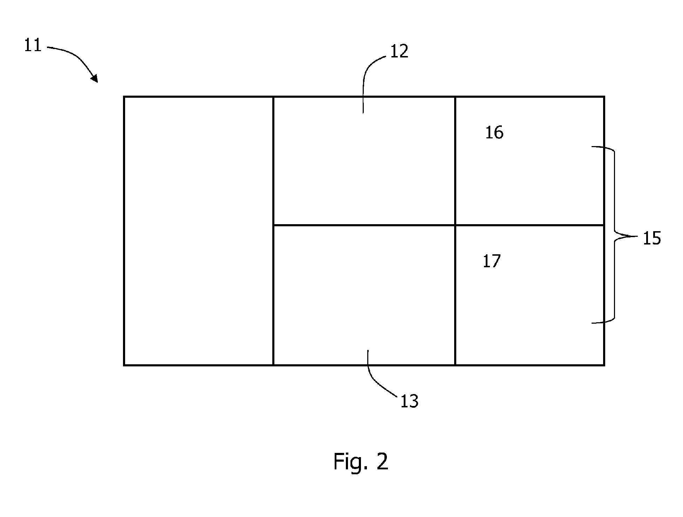

Das Falleingangsmodul und das Fallausgangsmodul sind bei einer zweckmäßigen Ausgestaltung für eine bidirektionale Kommunikation angelegt, sodass sich über dieses Modul sowohl Fallanfragen als auch Fallantworten erzeugen lassen. Ein dadurch gegebenes Fallmodul ist beispielsweise in Fig. 2 gezeigt.

Das Fallmodul 11 enthält ein Ein- und ein Ausgabeuntermodul 12 und 13. Diese Untermodule stellen die Nutzeroberfläche für den eingebenden oder lesenden Nutzer bereit. Sie enthalten insbesondere Software zur Dokumentenerzeugung, zum Einlesen von Dokumenten, zur Eingabe und Ausgabe von Daten, zum Prüfen einer Datenkonsistenz, sicherheitsspezifische Routinen, wie z.B. Passwortabfragen und dergleichen Identifikationsroutinen. Das Eingabeuntermodul 12 enthält weiterhin Registrierungsroutinen für das Fallmodul gegenüber der zentralen Servereinheit sowie Mittel zum Ausführen einer gesicherten Kommunikation über das Kommunikationsnetz, insbesondere Mittel zum Ver- und Entschlüsseln der mit der zentralen Servereinheit ausgetauschten Daten.

Weiterhin enthält das Fallmodul ein Anfrageuntermodul 14. Das Anfrageuntermodul 14 erzeugt aus den eingegebenen Fall- und Patientendaten die erwähnte Fallanfrage und gibt diese über das Ausgabeuntermodul an die zentrale Servereinheit aus.

Weiterhin ist bei dem Fallmodul ein Antwortuntermodul 15 vorgesehen. Das Antwortuntermodul erzeugt die oben erwähnten Fallantworten. Hierzu greift das Fallmodul über das Ein- und Ausgabeuntermodul auf eine lokale Datenbank des Endgerätes zurück, ermittelt dabei die aktuellen Ressourcen der Institution und gibt die Fallantwort in entsprechender Weise aus.

Das Antwortuntermodul 15 enthält mindestens eine Absageschnittstelle 16 und eine Zusageschnittstelle 17. Diese Schnittstellen enthalten die Fallantwort in ihrer kürzestmöglichen Form. Bei dem internen Matchingprozess wird dann das Resultat auf eine der beiden Schnittstellen geleitet und als Fallantwort "Zusage" oder "Absage" ausgegeben.

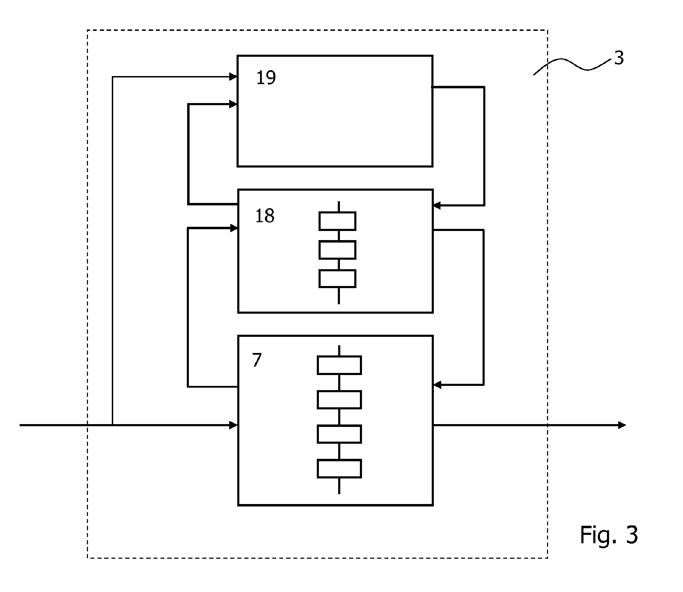

Fig. 3 zeigt grundlegende Komponenten zur Fallvermittlung, die innerhalb der zentralen Servereinheit 3 angeordnet sind bzw. von der zentralen Servereinheit 3 betrieben werden. Die zentrale Komponente der Fallvermittlung ist der bereits erwähnte Fallvermittlungsalgorithmus 7. Bei diesem handelt es sich um ein hard- und/oder softwareimplementiertes programmtechnisches Mittel.

Der Fallvermittlungsalgorithmus führt die eigentliche Vermittlung zwischen dem ersten Endgerät und dem zweiten Endgerät aus. Der Fallvermittlungsalgorithmus ist im hier gegebenen Beispiel mit einem Fallablaufsalgorithmus 18 verknüpft. Der hard- oder softwareimplementierte Fallablaufsalgorithmus 18 stellt grundlegende Ablaufpläne für komplexe Behandlungsabläufe bereit. Beide Algorithmen sind mit einer Falldatenbank 19 verknüpft, greifen auf die dort abgelegten Daten zu bzw. legen in der Falldatenbank Daten ab.

Der Fallvermittlungsalgorithmus 7 dient der Vermittlung der Fallanfrage. Der Fallablaufsalgorithmus ergänzend dazu eine Steuerung des Fallvermittlungsalgorithmus dar. Er sichert, dass die medizinischen Behandlungsschritte zum richtigen Zeitpunkt, unter den angemessenen Randbedingungen und vor allem in der richtigen Reihenfolge ausgeführt werden. Der Fallablaufsalgorithmus gewährleistet somit, dass zum richtigen Zeitpunkt die richtigen Fallvermittlungen ausgeführt werden und somit zeitraubende und unnötige Fehlvermittlungen vermieden werden.

Grundlage für diese Vermittlungskontrolle ist die Falldatenbank. Die Falldatenbank speichert den gesamten Behandlungsverlauf des individuellen Patienten und stellt somit seine Krankengeschichte jedenfalls in dem Umfang bereit, wie diese durch das Verfahren zur Fallvermittlung bereits bearbeitet worden ist.

Im einfachsten Fall erfolgt das Zusammenwirken des Fallvermittlungsalgorithmus, des Fallablaufsalgorithmus und der Falldatenbank in der folgenden Weise:

Die Fallanfrage wird zunächst dem Fallvermittlungsalgorithmus übergeben. Die Ausführung des Fallvermittlungsalgorithmus wird so lange ausgesetzt, bis der Fallablaufsalgorithmus bestätigt hat, dass der zu vermittelnde Behandlungsschritt tatsächlich aktuell zur Ausführung ansteht, dass dieser nicht schon ausgeführt wurde und/oder dass kein Behandlungsschritt übersprungen wird. Hierzu vergleicht der Fallablaufsalgorithmus den real nun anliegenden Behandlungsschritt mit einem vorgegebenen Behandlungsplan. Der Behandlungsplan wird aus der Falldatenbank abgerufen. Dieser enthält die individuell bereits ausgeführten Behandlungsschritte, die allgemein auszuführenden Behandlungsschritte in der richtigen Reihenfolge und mögliche individuell notwendigen und zusätzlich eingegebenen Abweichungen davon. Im Ergebnis eines positiven, d.h. übereinstimmenden Vergleichsresultates wird der Fallvermittlungsalgorithmus aus dem Wartezustand in den ausführenden Zustand versetzt und die Fallvermittlung wird ausgeführt.

Das Zusammenwirken des Fallvermittlungsalgorithmus mit dem Fallablaufsalgorithmus und der Falldatenbank ermöglicht somit eine automatisierte Behandlungsführung des einzelnen medizinischen Falles und eine Koordination mehrerer an dem Kommunikationsnetzwerk angeschlossener medizinischer Institutionen über deren entsprechenden Endgeräte.

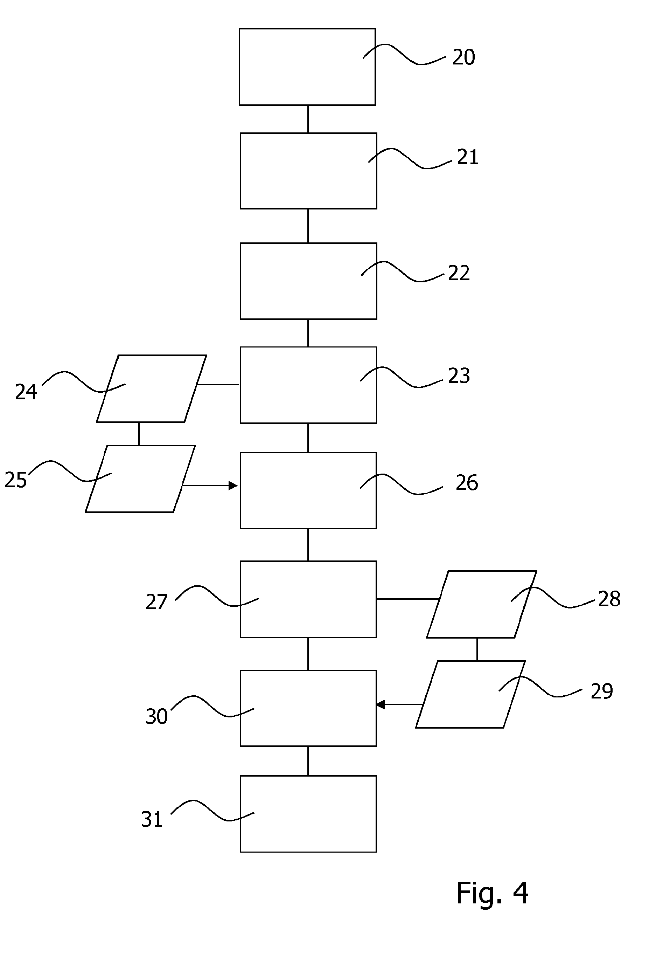

Fig. 4 zeigt einen beispielhaften Gesamtablaufplan des erfindungsgemäßen Verfahrens im Überblick. Einzelne Verfahrensschritte können dabei je nach Erfordernis und im Rahmen fachmännischen Handelns ohne weiteres angepasst oder verändert werden.

Das Verfahren beginnt mit einem Verfahrensschritt der Aufnahme von Einzeldaten 20. Dieser Verfahrensschritt beinhaltet zum einen die Aufnahme sämtlicher medizinischer Daten eines Patienten und zum anderen die Angabe der anstehenden nächsten Behandlungsschritte. In einem nächsten Schritt erfolgt sodann das Erzeugen der Fallanfrage 21. Dieses Erzeugen erfolgt in der oben angegebenen Weise. In einem Übermittlungsschritt 22 wird die Fallanfrage an die zentrale Servereinheit übertragen. Diese Übertragung erfolgt über ein Kommunikationsnetzwerk, insbesondere das Internet. In einem nächsten Schritt 23 wird die Fallanfrage an den Fallvermittlungsalgorithmus übergeben. Hierzu wird der Fallvermittlungsalgorithmus in einem Aufrufeschritt 24 aus einem Speicher der zentralen Servereinheit aufgerufen. In Verbindung damit erfolgt ein Verknüpfen 25 zwischen dem Fallvermittlungsalgorithmus und dem ebenfalls im Speicher der zentralen Servereinheit vorliegenden Fallablaufsalgorithmus.

In einem darauf folgenden Schritt 26 wird die Fallanfrage an eine Reihe von zweiten Endgeräten gesendet. Dies erfolgt wiederum über das Kommunikationsnetz. In den zweiten Endgeräten wird in einem Schritt 27 die Fallanfrage verarbeitet und in Form einer Fallantwort beantwortet. Die Fallantwort wird über die zentrale Servereinheit und dort insbesondere wieder dem Fallvermittlungsalgorithmus in einem Schritt 28 unterzogen, wobei abermals ein Verknüpfungsschritt 29 mit dem Fallablaufsalgorithmus erfolgt. Die Fallantwort wird sodann in einem Schritt 30 an das erste Endgerät zurückgesendet oder von dem ersten Endgerät von der zentralen Servereinheit herunter geladen. Die Fallantwort wird sodann in einem Schritt 31 an dem ersten Endgerät ausgegeben.

Es ist einsichtig, dass zu jeder Fallanfrage mehrere Fallantworten von mehreren zweiten Endgeräten an das erste Endgerät übermittelt werden können. Der Nutzer des ersten Endgerätes erhält dadurch einen Überblick über die vorhandenen Möglichkeiten einer nachfolgenden Patientenversorgung und kann somit entsprechend planen und auswählen. Es ist ebenfalls einsichtig, dass an jedem der zweiten Endgeräte auch mehrere Fallanfragen eingehen können. Sowohl die Institution, die Fallanfragen ausgibt, als auch die Institution, die Fallanfragen entgegen nimmt, erhält somit einen Überblick über das Angebot und den Bedarf an medizinischen Dienstleistungen.

Bei einer weiteren Ausgestaltung des Verfahrens kann die zentrale Servereinheit auch aus mehreren Servern aufgebaut sein, die miteinander kommunizieren. Die einzelnen Server sind dabei zweckmäßigerweise regional an mehreren Standorten verteilt und decken einen Teilbereich des Kommunikationsnetzes ab. Sie verfügen somit über einen regionalen Einzugsbereich, innerhalb dem die medizinischen Behandlungsschritte vermittelt werden. Innerhalb der Fallvermittlungsalgorithmen in jedem der regionalen Server kann dann vorgesehen sein, dass eine Fallanfrage dann zu einem übergeordneten Server oder zu einem anderen regionalen Server weiter geleitet wird, wenn die Fallantworten im regionalen Einzugsbereich sämtlich abschlägig beschieden werden. Derselbe Vorgang kann dann auch aktiviert werden, wenn die Fallanfrage auf eine medizinische Behandlung zielt, die ein spezielles Fachwissen voraussetzt, das nur an einer überregionalen Institution vorhanden ist. Dies kann beispielsweise bei komplizierten Behandlungen wie Transplantationen oder Herzoperationen der Fall sein.

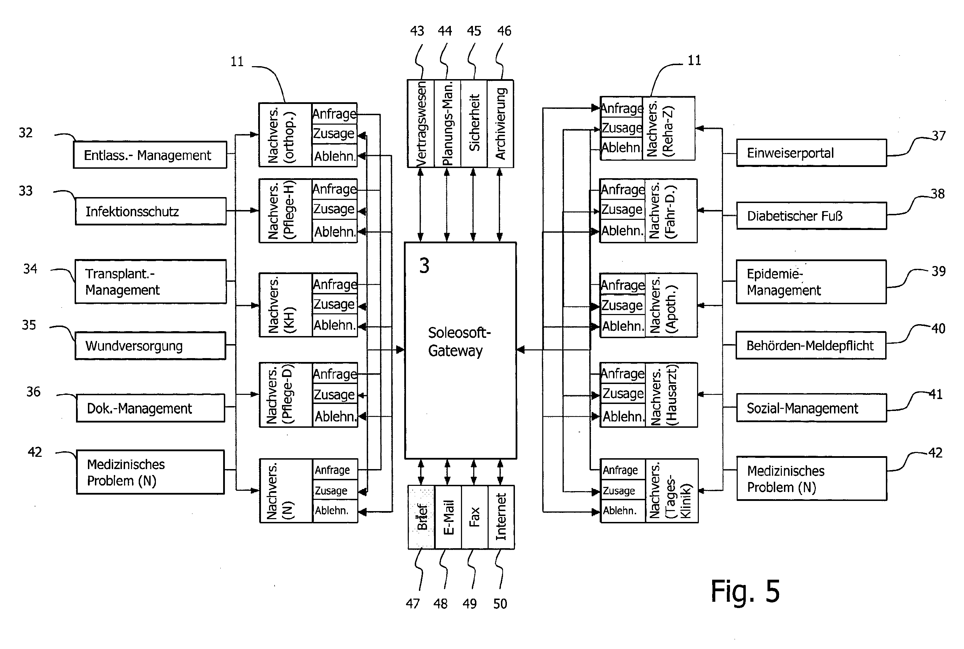

Fig. 5 zeigt in einer Übersichtsdarstellung eine Ausführungsform des erfindungsgemäßen Verfahrens, bei dem die erwähnten Vermittlungsschritte mit so genannten Fallmanagementsystemen gekoppelt sind. Bei diesen Fallmanagementsystemen handelt es sich um jeweils den einzelnen Institutionen zugeordnete Mittel, mit denen sich spezielle medizinische Probleme einzelfallbezogen in ihrem Behandlungsablauf planen und organisieren lassen. Dies können insbesondere Krankenhaus- oder Praxisorganisationssysteme sein.

Diese Mittel sind entweder softwareartig oder hardwareartig in den entsprechenden Endgeräten der einzelnen Institutionen implementiert. In dem hier vorliegenden Beispiel sind dies ein Entlassungsmanagement 32, ein Infektionsschutzmanagement 33, ein Transplantationsmanagement 34, ein Wundversorgungsmanagement 35, ein Dokumentationsmanagement 36, ein Management für ein Einweiserportal 37, ein Diabetisches Management 38, ein Epidemiemanagement 39, ein Meldemanagement 40 und ein Sozialmanagement 41 sowie weitere Managementsysteme 42.

Diese Managementsysteme steuern die Behandlungsabläufe vor Ort und somit in der Umgebung des jeweiligen Endgerätes. Sie sind jeweils mit einem Fallmodul 11 verknüpft, das ebenfalls wie beschrieben auf dem Endgerät implementiert ist. Die Fallanfrage läuft nun so ab, dass das jeweilige Managementsystem aktuelle Patienten- und Behandlungsdaten automatisiert an das Fallmodul übergibt, woraufhin dieses dann die Fallanfrage generiert und an die zentrale Servereinheit 3 übergibt. Dem Managementsystem vor Ort korrespondieren in einem solchen Fall der übergreifende Fallvermittlungsalgorithmus und der Fallablaufsalgorithmus in der zentralen Servereinheit. Dadurch greifen diese an sich lokal eingerichteten Managementsysteme auf das nicht lokale Kommunikationsnetz zu.

Fig. 5 zeigt weitere Zugangs- und Interaktionsmöglichkeiten mit der zentralen Servereinheit. Diese dienen vor allem einer externen Parametersetzung für die Interaktion zwischen der zentralen Servereinheit und den Fallmodulen der Engerätemenge und können von dazu berechtigten Nutzern bedient werden. Vorgesehen ist im hier gegebenen Beispiel insbesondere eine mit einem Vertragswesen gekoppelte Schnittstelle 43. Diese erlaubt es, gewisse Freischalte- oder Zugangsoptionen für die Fallmodule an den Abschluss eines Vertrages oder einer Zertifizierung zu knüpfen. Eine Schnittstelle 44 für ein Planungsmanagement ermöglicht es, gewisse Einschränkungen oder Umstellungen in der Organisation der Fallmodule auf den Endgeräten innerhalb der zentralen Servereinheit vorzunehmen und somit globale Parameter in der Fallbearbeitung zu setzen. Eine Sicherheitsschnittstelle 45 ermöglicht das Setzen von allgemeinen Sicherheitsparametern, das Implementieren von Verschlüsselungen, das Zuweisen von Zugangsberechtigungen und dergleichen Operationen. Eine Archivierungsschnittstelle 46 stellt eine Verknüpfung zu einer zugeordneten Archivierungsdatenbank bereit.

Kommunikationen mit der zentralen Servereinheit können natürlich nicht nur über die Interaktion mit den Fallmodulen erfolgen. Möglich sind hier auch das Einsenden von Briefen 47, die Kommunikation via e-Mail 48, Fax 49 oder über ein TCP/IP-Protokoll über den Aufruf einer Website 50. Letzteres ermöglicht insbesondere einen umstandslosen und einfachen individuellen Registrierungsvorgang für die jeweiligen Fallmodule bzw. für deren Nutzer.

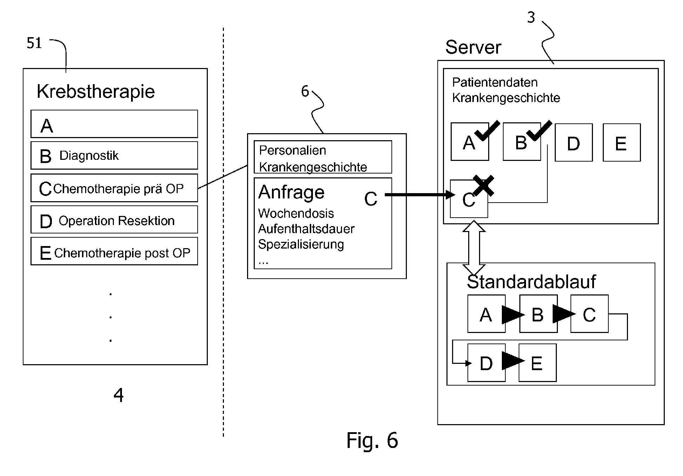

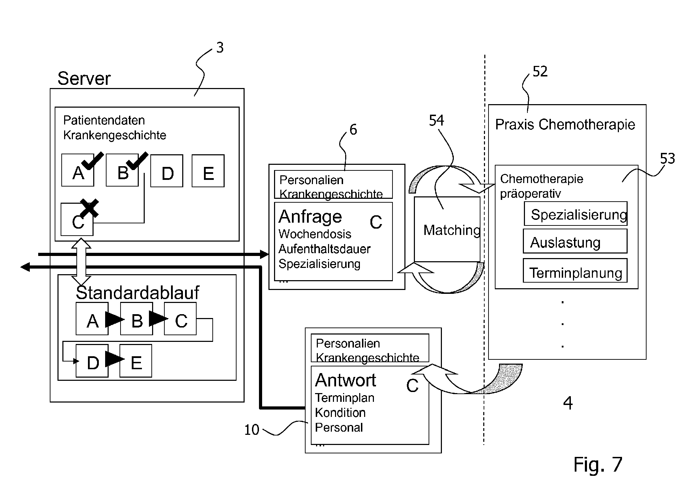

Anhand der Figuren 6 und 7 soll nachfolgend anhand eines medizinischen Einzelfalles das Zusammenwirken zwischen einerseits einem Managementsystem und der Generierung einer Fallanfrage sowie deren Verarbeitung auf der zentralen Servereinheit einerseits und andererseits zwischen der zentralen Servereinheit und einem Endgerät in einer beispielhaften medizinischen Institution zum Generieren einer Fallantwort dargestellt werden.

Bei dem nachfolgenden Beispiel wird von einem medizinischen Einzelfall ausgegangen, bei dem ein Patient einer Krebstherapie unterzogen wird. Die Krebstherapie besteht aus mehreren Schritten A bis E. Bei dem hier vorliegenden Beispiel sind bereits die Schritte A und B ausgeführt worden. Es steht nun ein Schritt C an, der eine präoperative Chemotherapie beinhaltet. Nach der präoperativen Chemotherapie ist im weiteren Verlauf ein Schritt D vorgesehen. Dieser beinhaltet die operative Resektion des Tumorgewebes. Abschließend ist ein Schritt E geplant, der eine postoperative Chemotherapie beinhaltet.

Der Fall des Patienten befindet sich im hier vorliegenden Beispiel zunächst im Bereich der Praxis seines Hausarztes. Der Hausarzt aber führt eine präoperative Chemotherapie nicht aus, somit wird eine schnellstmögliche Überweisung des Patienten eine chemotherapeutische Praxis erforderlich. Die Vermittlung des Patienten von der Hausarztpraxis zu der chemotherapeutischen Praxis erfolgt unter Rückgriff auf das Fallmanagmentsystem "Krebstherapie" in der Praxis des Hausarztes einerseits und unter Verwendung des vorhergehend erläuterten Vermittlungsverfahrens andererseits mit den nachfolgend erläuterten Schritten.

Auf dem Endgerät in der Hausarztpraxis ist ein Managementsystem 51 für Krebstherapie installiert. Dieses ist mit Patientendaten verknüpft. Die Patientendaten enthalten insbesondere die Information, dass ein Diagnostikschritt B bereits abgeschlossen ist. Auf dem Endgerät in der Hausarztpraxis wird nun eine Fallanfrage 6 ausgelöst, die sich auf den Schritt C der präoperativen Chemotherapie des Managementsystems bezieht. Die Fallanfrage enthält die Personalien des Patienten, seine Krankengeschichte sowie die erforderliche Wochendosis, eine notwendige Aufenthaltsdauer, geforderte Spezialkenntnisse zur Fallbearbeitung und dergleichen Angaben. Die Fallanfrage wird an die zentrale Servereinheit 7 gesendet.

In der zentralen Servereinheit ist der bisherige Verlauf der Krankengeschichte des Patienten aus vorherigen Vermittlungsvorgängen bereits gespeichert. Ein dort wie beschrieben ablaufender Fallablaufsalgorithmus umfasst ebenso wie in dem Managementsystem in der Hausarztpraxis die notwendigen Behandlungsschritte A bis E. Die gespeicherte Krankengeschichte des Patienten weist aus, dass die Schritte A und B bereits ausgeführt und beendet worden sind. Die Anfrage auf den Schritt C ist somit formell korrekt und entspricht somit einem vorgegebenen Standardablauf. Somit kann die Fallanfrage dem Fallvermittlungsalgorithmus innerhalb der zentralen Servereinheit übergeben werden.

Der Fallvermittlungsalgorithmus leitet nun die Fallanfrage 6 an das Endgerät mindestens einer chemotherapeutischen Praxis 52 weiter. Die in der Anfrage enthaltenen Daten werden von dem dort installierten chemotherapeutischen Managementsystem 53 analysiert und einem Matchingprozess 54 unterzogen. Dies betrifft insbesondere Daten über die notwendige Art der Chemotherapie und die dafür vorhandenen Praxisressourcen, den Grad der Intensität der Therapie und den Grad der Auslastung der Praxis und dergleichen Daten mehr. Eine Absage wird in einem solchen Fall dann ausgegeben, wenn das Matching eine größtmögliche Abweichung zwischen den Daten der Fallanfrage und den Ressourcendaten im chemotherapeutischen Managementsystem aufweist.

Im Ergebnis wird auf dem zweiten Endgerät die Fallantwort 10 erzeugt. Diese ist eindeutig auf den Behandlungsschritt C bezogen und wird zu der zentralen Servereinheit 3 zurückgesendet und dort protokolliert. Dadurch werden die Patientendaten auf einen aktuellen Stand gesetzt. Die Fallantwort enthält in diesem Beispiel gleichzeitig noch nähere Angaben über einen Terminplan, gewisse Nebenbedingungen und Angaben über das zur Ausführung der Chemotherapie angewiesene Personal. Diese Fallantwort wird an dem Endgerät der Hausarztpraxis abgerufen und kann in dem dort installierten Managementsystem verarbeitet werden.

Das über das Kommunikationsnetzwerk ausgeführte Verfahren ermöglicht es somit, medizinische Problemstellungen, die eine gegenseitige Abstimmung verschiedener Instanzen erfordern elektronisch, gesichert und nachvollziehbar zu bearbeiten. Das Verfahren stellt wie beschrieben auch sicher, genau die richtigen Institutionen in der richtigen Reihenfolge angesprochen werden und auch nur auf genau die Information einen Zugriff erhalten, die für Sie bestimmt und notwendig sind.

Das elektronische Netzwerk wird über die standardmäßigen und zeitgemäßen Kommunikationswege dem Netzwerkteilnehmer bereitgestellt. Es ist mit den entsprechenden Sicherheitseinrichtungen, insbesondere Verschlüsselungen, ausgestattet, welche einen unberechtigten Datenzugriff bei der Übertragung verhindern. Die zentrale Servereinheit, an und von der der alle Daten ein- bzw. auslaufen, wird ebenfalls mit den Sicherheitsmitteln betrieben ist vor unberechtigtem Zugriff geschützt.

Bei dem Netzwerk ist außerdem vorgesehen, dass Schnittstellen verschiedenen bereits bestehenden Pflege-Systemen und/oder Krankenhaus-Informationssystem bzw. auch anderen Systemen ausgebildet sind, über die ein bidirektionaler Datenaustausch mit den Komponenten des hier beschriebenen Verfahrens erfolgen kann. Es ist dann möglich, einen vollständigen Informationsaustausch mit bereits bestehenden Systemen auszuführen.

Das gesamte Verfahren erfolgt automatisiert. Der erwähnte Registrierungsprozess der einzelnen Endgeräte und der darauf installierten Fallmodule ermöglicht eine Administrierung einzelner Nutzer und Teilnehmer und eine gezielte Kontrolle darüber, wem ein Zugang zu den Kommunikationsvorgängen im Netzwerk eingeräumt wird.

In einem weiteren Fallbeispiel soll nun ein so genanntes Entlassungsmanagement, hier am Beispiel einer Nachversorgung nach einer Beinoperation, beschrieben werden. Bei diesem Beispiel benötigt der Patient eine entsprechende Nachsorge und Hilfsmittel zur Bewegung.

In einem solchen Fall sind auf einem im Krankenhaus befindlichen Endgerät Mittel für ein so genanntes Entlassungsmanagement installiert. Das Entlassungsmanagement stellt sicher, dass alle Nachversorger über den Vorgang des Patienten so informiert werden, dass eine zeitnahe Erfassung des Patienten erfolgen kann.

Das Entlassungsmanagement greift nun auf das erfindungsgemäße Verfahren zurück. In dem entsprechenden Kommunikationsnetzwerk sind neben dem Krankenhaus eine Reihe von Nachversorgern angemeldet. Jeder dieser Nachversorger ist entsprechend registriert. Er bekommt jeweils von dem Krankenhaus elektronisch eine Fallanfrage darüber, dass ein Patient für zur Nachsorge übergeben werden soll. Jeder der registrierten Nachversorger kann jetzt online über das Netzwerk den Patienten annehmen oder ablehnen. Die Entscheidung, wer die Nachversorgung auswählt, wird direkt am Endgerät des Krankenhauses zur Verfügung gestellt. Am Endgerät des Krankenhauses liegt damit sofort eine Information darüber an, ob der Patient entlassen werden darf oder nicht.

Am Endgerät des Krankenhauses kann gegebenenfalls unter mehreren Nachsorgern ausgewählt werden. Die Entlassung des Patienten wird dabei zeitnah umgesetzt. Diese Auswahlprozedur kann durch optische oder akustische Signale unterstützt sein. Dies ist beispielsweise dann der Fall, wenn eine Fallantwort einer der Nachversorger signalisiert, dass der betreffende Patient praktisch sofort überstellt werden kann. In einem solchen Fall wird am Endgerät des Krankenhauses ein optisches Signal ausgegeben und der Patient kann zur vereinbarten Zeit entlassen werden.

Dieser Vorgang kann mit einer Rückmeldeprozedur verknüpft sein. In dem hier gegebenen Beispiel wird nach einer festgelegten Zeitdauer, beispielsweise 48 nach der Entlassung, automatisch eine Rückmeldung des jeweiligen Nachversorgers eingefordert. Sofern die Rückmeldung am Endgerät des Krankenhauses eingetroffen ist, wird dort Entlassung als Vorgang abgeschlossen und archiviert.

Das nachfolgend genannte weitere Beispiel betrifft ein Pflegemanagement:
Bei diesem Beispiel wird von einem Unfall eines Pflegepatienten in einem Pflegeheim ausgegangen, bei dem eine Fraktur festgestellt wird. Daher ist der Patient so schnell wie möglich in ein Krankenhaus überweisen.
Bei dem hier vorliegenden Verfahren wird somit eine Fallanfrage an Reihe von Krankenhäusern erzeugt. Als Fallantwort wird daraufhin von jedem angesprochenen Endgerät jedes einzelnen Krankenhauses eine aktuelle Bettenkapazität zurückgegeben. Diese wird auf dem Endgerät im Pflegeheim angezeigt. Das Pflegeheim verfügt damit online über die aktuelle Bettenkapazität jedes einzelnen Krankenhauses innerhalb einer bestimmten Region. Der Krankenwagentransport kann den Patienten direkt in genau das Krankenhaus transportieren, in dem freie Betten verfügbar sind und die Behandlung des Patienten schnellstmöglich begonnen werden kann. Außerdem liegt auf dem Endgerät des Krankenhauses bereits sie Fallanfrage vor. Die Einlieferung des Patienten ist also bekannt und es kann die elektronische Krankenakte im Vorfeld schon dem Krankenhaus zugesendet werden bzw. ein Zugriff auf die Akte des Patienten kann online erfolgen.

Nach der Behandlung des Patienten kann der oben beschriebene Prozess des Entlassungsmanagements wie im vorhergehenden Fallbeispiel ausgeführt werden. In dem hier gegebenen Fallbeispiel wird der Patient wieder in das Pflegeheim rücküberwiesen. Dort wird an das Endgerät des Pflegeheims die komplette Krankenakte elektronisch übermittelt und in das Datenbanksystem des Pflegeheims überspielt.

Die Erfindung wurde anhand von Ausführungsbeispielen erläutert. Im Rahmen fachmännischen Handelns sind weitere Ausführungsformen möglich. Diese ergeben sich insbesondere aus den Unteransprüchen.

### Bezugszeichenliste

- 1: Kommunikationsnetzwerk
- 2: verteilte Endgerätemenge
- 3: zentrale Servereinheit
- 4: erstes Endgerät
- 5: Falleingangsmodul
- 6: Fallanfrage
- 7: Fallvermittlungsalgorithmus
- 8: zweites Endgerät
- 9: Fallausgangsmodul
- 10: Fallantwort
- 12: Eingabeuntermodul
- 13: Ausgabeuntermodul
- 14: Anfrageuntermodul
- 15: Antwortuntermodul
- 16: Absageschnittstelle
- 17: Zusageschnittstelle
- 18: Fallablaufsalgorithmus
- 19: Falldatenbank
- 20: Aufnahme Einzeldaten
- 21: Erzeugen Fallanfrage
- 22: Übermittlungsschritt
- 23: Übergabe an Fallvermittlungsalgorithmus
- 24: Aufrufeschritt
- 25: Verknüpfen Fallvermittlungsalgorithmus - Fallablaufsalgorithmus
- 26: Senden Fallanfrage an zweite Endgeräte
- 27: Verarbeiten Fallanfrage an zweitem Endgerät
- 28: Vermitteln Fallantwort über Fallvermittlungsalgorithmus
- 29: Verknüpfen Fallvermittlungsalgorithmus - Fallablaufsalgorithmus
- 30: Rücksenden Fallantwort an erstes Endgerät
- 31: Ausgabe Fallantwort an erstem Endgerät
- 32: Entlassungsmanagement
- 33: Infektionsschutzmanagement
- 34: Transplantationsmanagement
- 35: Wundversorgungsmanagement
- 36: Dokumentationsmanagement
- 37: Management für Einweiserportal
- 38: Diabetisches Management
- 39: Epidemiemanagement
- 40: Meldemanagement
- 41: Sozialmanagement
- 42: weitere Managementsysteme
- 43: Schnittstelle Vertragswesen
- 44: Schnittstelle Planungsmanagement
- 45: Sicherheitsschnittstelle
- 46: Archivierungsschnittstelle
- 47: Brief
- 48: e-Mail
- 49: Fax
- 50: TCP/IP; Website
- 51: Krebstherapiemanagement

## Patentansprüche

1. Verfahren zur Vermittlung von einzelfallbezogenen, institutionsübergreifenden Behandlungsschritten bei einer aus einer Vielzahl von individuellen Einzelpatienten bestehenden Patientenmenge,
unter Nutzung eines Kommunikationsnetzwerkes (1) mit einer verteilten Endgerätemenge (2) und einer zentralen Servereinheit (3),
mit folgenden Verfahrensschritten:
- Erfassen fall- und personenspezifischer Einzeldaten an mindestens einem ersten Endgerät (4) mittels eines auf dem Endgerät betriebenen Falleingangsmoduls (5),
- Erzeugen einer Fallanfrage (6) durch das Falleingangsmodul und übermitteln der Fallanfrage an die zentrale Servereinheit,
- Übergeben der Fallanfrage an einen von der zentralen Servereinheit betriebenen Fallvermittlungsalgorithmus (7),
- Ausgabe der Fallanfrage von der zentralen Servereinheit an mindestens ein durch den Fallvermittlungsalgorithmus ausgewähltes zweites Endgerät (8),
- Verarbeiten der Fallanfrage auf dem mindestens einen zweiten Endgerät in einem dort betriebenen Fallausgangsmodul (9),
- Rückleiten einer Fallantwort (10) von dem zweiten Endgerät an die zentrale Servereinheit,
- Weiterleiten der Fallantwort von der zentralen Servereinheit auf das erste Endgerät.

2. Verfahren nach Anspruch 1,
**dadurch gekennzeichnet, dass**
das Falleingangsmodul (5) und das Fallausgangsmodul (9) gleichartig auf jedem Endgerät als jeweils ein Fallmodul (11) ausgebildet sind, wobei das Fallmodul jeweils über ein Eingabe- und ein Ausgabeuntermodul (12, 13) für einen Nutzer, ein Anfrageuntermodul (14) und ein Antwortuntermodul (15) in Kommunikation mit der zentralen Servereinheit (3) betrieben wird.

3. Verfahren nach Anspruch 2,
**dadurch gekennzeichnet, dass**
bei dem Antwortuntermodul (15) über mindestens einen Absageausgang (16) und einen Zusagenausgang (17) ein Ausgeben einer positiven oder negativen Antwort auf die eingehende Fallanfrage erfolgt.

4. Verfahren nach einem der vorhergehenden Ansprüche,
**dadurch gekennzeichnet, dass**
der auf der zentralen Servereinheit (3) ablaufende Fallvermittlungsalgorithmus (7) über einen Fallablaufsalgorithmus (18) einen Zugriff auf eine Falldatenbank (19) ausführt, wobei dadurch eine auf einen jeweiligen Einzelfall bezogene automatisierte Ablaufplanung der Abfolge einzelner Behandlungsschritte ausgeführt wird.

5. Verfahren nach einem der vorhergehenden Ansprüche,
**dadurch gekennzeichnet, dass**
jedes Falleingangsmodul (5), Fallausgangsmodul (9) und/oder Fallmodul (11) über einen Registrierungsvorgang an der zentralen Servereinheit (3) angemeldet wird, wobei eine wahlweise Sperrung und/oder Inaktivierung jedes der Module ausführbar ist.

6. Verfahren nach einem der vorhergehenden Ansprüche,
**dadurch gekennzeichnet, dass**
die zentrale Servereinheit (3) einen Datenbankzugriff auf Vertragsdaten, Sicherheitsdaten, Archivierungsdaten und dergleichen weitere Daten ausführt.

7. Verfahren nach einem der vorhergehenden Ansprüche,
**dadurch gekennzeichnet, dass**
das Falleingangsmodul (5), das Fallausgangsmodul (9) und/oder das Fallmodul (11) in Verbindung mit einem externen Fallmanagementsystem betrieben werden, wobei das Fallmanagementsystem ein automatisiertes Aktivieren des jeweiligen Falleingangsmoduls, des Fallausgangsmoduls und/oder des Fallmoduls ausführt.

8. Anordnung zur Vermittlung von auf einen jeweiligen Einzelfall bezogenen, institutionsübergreifenden Behandlungsschritten bei einer aus einer Vielzahl von individuellen Einzelpatienten bestehenden Patientenmenge,
unter Nutzung eines Kommunikationsnetzwerkes (1) mit einer verteilten Endgerätemenge (2) und einer zentralen Servereinheit (3),
**gekennzeichnet durch**
auf jedem Endgerät der Endgerätemenge angeordnete programmtechnische Mittel für mindestens ein Fallmodul (11) und programmtechnische Mittel für einen auf der zentralen Servereinheit ablauffähigen Fallvermittlungsalgorithmus (7) zum Eingeben, Weiterleiten und Vermitteln einer individuellen medizinischen Fallanfrage (6) zwischen verschiedenen Anbietern und Behandlungsstationen.

9. Anordnung nach Anspruch 8,
**dadurch gekennzeichnet, dass**
das Fallmodul jeweils ein Eingabe- und ein Ausgabeuntermodul (12, 13) für einen Nutzer, ein Anfrageuntermodul (14) und ein Antwortuntermodul (15) in Kommunikation mit der zentralen Servereinheit aufweist, wobei bei dem Antwortuntermodul (15) über mindestens eine Absageschnittstelle (14) und eine Zusageschnittstelle (17) ein Ausgeben einer positiven oder negativen Antwort auf die eingehende Fallanfrage ausführbar ist.

10. Anordnung nach einem der Ansprüche 8 oder 9,
**dadurch gekennzeichnet, dass**
das Fallmodul (11) jeweils an der zentralen Servereinheit (3) registriert ist, wobei eine wahlweise Sperrung und/oder Inaktivierung des Fallmoduls ausführbar ist.
